# EUROPEAN PATENT APPLICATION

(11) **EP 2 930 510 A1**
(43) Date of publication of application: **14.10.2015**
(21) Application number: 14305531.7
(22) Date of filing: 10.04.2014
(51) Int. Cl.: G01N 33/28

(54) **System and method for measuring free and dissolved gasses in drilling fluids**

(71) Applicant: Geoservices Equipements, 95700 Roissy en France (FR)
(72) Inventor: Breviere, Jerome, 95971 Roissy en France (FR); Kasprzykowski, Pawel, 95971 Roissy en France (FR)
(74) Representative: Rzaniak, Martin

(57) **Abstract**

A method is disclosed and performed by collecting, by a volumetric sampler (50), a known volume of a sample from drilling fluid (28) exiting a well bore (12) penetrating a formation (14), the sample having a free gas and a dissolved gas. A first quantity of the free gas entrained in the sample is measured by a free gas detection system (52). The sample is transferred to a dissolved gas detection system (54) after the free gas in the sample is measured. The method is further performed by measuring a second quantity of the dissolved gas in the sample by the dissolved gas detection system. A total amount of one or more certain type of gas in a formation is estimated based on the first and second quantities.

## Description

### BACKGROUND

Well bores are drilled to locate and produce hydrocarbons from geologic formations. A down hole drilling tool with a bit at an end thereof is advanced into the geologic formation to form a well bore. As the drilling tool is advanced, a drilling mud is pumped through and out of the drilling tool to cool the drilling tool and carry away cuttings. The drilling mud additionally forms a mud cake that lines the well bore. The drilling mud is cycled through the wellbore, being pumped into the wellbore from the surface, encountering fluids and gasses within the wellbore and traveling back up to the surface. During the drilling process, CO₂ is monitored. Too much CO₂ in formation fluid can generate additional costs in production and lead to the abandonment of operations.

Formation evaluation often employs fluid from the formation drawn into the down hole tool for testing and/or sampling. Various devices, such as probes, are extended from the down hole tool to establish fluid communication with the formation surrounding the well bore and to draw fluid into the down hole tool. Wireline tools such as repeat formation testers (RFT) and modular dynamic formation testers (MDT) extend the tester along a wire down hole into the wellbore. Laboratory tests are performed on formation fluid samples retrieved by these wireline tools. Most down hole tools do not allow for precise estimation of CO₂ present in a geologic formation, simply measuring free gasses present in drilling mud.

A method, described in U.S. 7,666,679 to Herzhaft et al., suggests in-line measuring of CO₂. Herzhaft et al. teaches creating a specific installation on a closed part of a flow line. However, mechanical and safety reasons present challenges to this method.

Some versions of in-line CO₂ measuring devices are installed on an open portion of the flow line of the drilling fluid. This open sampling allows escape of free CO₂ to the atmosphere inhibiting measurement of CO₂ within the drilling mud to a fraction of the CO₂ coming from the formation and resulting in underestimated CO₂ readings. Open line solutions also present the possibility of pollution by mud additives and formation rocks, such as carbonates. Contamination by carbonate materials leads to overestimation of CO₂ readings for the drilling mud.

### SUMMARY

In one version, the present disclosure describes a mud gas analyzer having a volumetric sampler collecting a known volume of a sample of drilling fluid having a free gas and a dissolved gas, a free gas detection system in fluid communication with the volumetric sample, and a dissolved gas detection system in fluid communication with the volumetric sampler. The volumetric sampler filters the known volume of the sample and releases the free gas entrained with the known volume of the sample. The free gas detection system receives the free gas from the volumetric sampler and has at least one sensor to determine a first quantity of the free gas. The dissolved gas detection system has a reaction chamber in fluid communication with the volumetric sampler, an acidification system in fluid communication with the reaction chamber, and a sensor connected to the reaction chamber. The reaction chamber has a first end, a second end, a sidewall extending between the first end and the second end, an inlet formed within the sidewall, and an outlet formed within the sidewall. The reaction chamber receives the known volume of the sample with the dissolved gas entrained therein via a first inlet of the inlet in fluid communication with the volumetric sampler. The acidification system has an acid tank and an injection system. The acid tank has an inlet and an outlet. The injection system is connected to the acid tank and injects an acid, through the outlet, into the reaction chamber through an inlet of the reaction chamber. The sensor detects a second quantity of the dissolved gas released from the known volume of the sample within the reaction chamber.

In one embodiment, a method is described as collecting, by a volumetric sampler, a known volume of a sample from drilling fluid exiting a wellbore penetrating a formation. The sample has a free gas and a dissolved gas. A first quantity of the free gas entrained in the sample is measured. The sample is transferred to a dissolved gas detection system after the free gas entrained in the sample is measured. The method is further performed by measuring a second quantity of the dissolved gas in the sample by the dissolved gas detection system. A total amount of a certain type of gas in a formation is estimated based on the first and second quantities.

In another version, the present disclosure describes a mud gas analyzer having a volumetric sampler collecting a known volume of a sample of drilling fluid having a free gas and a dissolved gas, a free gas detection system in fluid communication with the volumetric sampler, an ionic chromatography system in fluid communication with the volumetric sampler, and a sensor in fluid communication with the ionic chromatography system. The volumetric sampler filters the known volume of the sample and releases the free gas entrained within the known volume of the sample. The free gas detection system receives the free gas from the volumetric sampler and has at least one sensor to determine a first quantity of the first free gas. The ionic chromatography system has a sensor and determines a concentration of predetermined ions within the known volume of the sample.

### BRIEF DESCRIPTION OF DRAWINGS

Certain embodiments of the inventive concepts will hereafter be described with reference to the accompanying drawings, wherein like reference numerals denote like elements. It should be understood, however, that the accompanying figures illustrate the various implementations described herein and are not meant to limit the scope of the various technologies described herein.
Figure 1 is a schematic view of some embodiments of a well site in accordance with the present disclosure.
Figure 2 is a schematic view of some embodiments of a mud gas analyzer constructed in accordance with the present disclosure.
Figure 3 is a schematic view of a computer system electrically coupled to the mud gas analyzer of Figure 2.
Figure 4 is a schematic view of a mud gas analyzer constructed in accordance with some embodiments of the present disclosure.

### DETAILED DESCRIPTION

Specific embodiments of the present disclosure will now be described in detail with reference to the accompanying drawings. Further, in the following detailed description of embodiments of the present disclosure, numerous specific details are set forth in order to provide a more thorough understanding of the disclosure. However, it will be apparent to one of ordinary skill in the art that the embodiments disclosed herein may be practiced without these specific details. In other instances, well-known features have not been described in detail to avoid unnecessarily complicating the description.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concept. This description should be read to include one or at least one and the singular also includes the plural unless otherwise stated.

The terminology and phraseology used herein is for descriptive purposes and should not be construed as limiting in scope. Language such as "including," "comprising," "having," "containing," or "involving," and variations thereof, is intended to be broad and encompass the subject matter listed thereafter, equivalents, and additional subject matter not recited or inherently present therein.

As used herein any references to "one embodiment" or "an embodiment" means that a particular element, feature, structure, or characteristic described in connection with the embodiment is included in at least one embodiment. The appearances of the phrase "in one embodiment" in various places in the specification may not refer to the same embodiment.

Some aspects of the disclosure are applicable throughout the life of a wellbore including, but not limited to, during drilling, logging, drill stem testing, fracturing, stimulation, completion, cementing, and production.

When CO₂ from a geological formation enters a wellbore and is mixed with drilling fluid, the CO₂ may reach a surface of a well in two different forms, CO₂ dissolved in the mud and a free/gaseous CO₂. The gaseous CO₂ reacts with the drilling fluid pumped into the wellbore and may be partially dissolved following Henry's law, p = K_{H} · [CO₂]. In the referenced equation, "p" is a partial pressure of CO₂ in the gas from above the solution, i.e. CO₂ in addition to the drilling fluid. "[CO₂]" is [CO_{2aqueous}] - concentration of CO_{2aqueous}. "K_{H}" is the Henry's law constant and depends on the drilling fluid type and temperature. The reaction of CO₂ gas with the drilling mud produces the CO₂ (aqueous). Equilibrium between CO₂ dissolved within the drilling fluid and free CO₂ may be established by CO₂ (aq) + H₂O ↔ H₂CO₃. Two carbonates denoting the presence of dissolved CO₂ within a drilling fluid are HCO₃ and CO₃²⁻. These carbonates are formed through the following reactions: H₂CO₃ ↔ H⁺ + HCO₃⁻ and HCO₃⁻ ↔ H⁺ + CO₃²⁻, respectively. A dissociation constant, pKa for the HCO₃⁻ reaction is 6.4 and for the CO₃²⁻ reaction is 10.3. Free/gaseous CO₂ within the drilling fluid may be measured after a degassing process such as agitation, which releases the free CO₂. Dissolved CO₂ within the drilling fluid may be determined by releasing the dissolved CO₂ or by measuring ion concentrations, such as carbonates, within the drilling fluid. The dissolved CO₂ may be released by an acidification process, to directly measure the dissolved CO₂. An amount of CO₂ dissolved in the drilling fluid may also be determined by knowing equilibrium conditions and carbonate ion concentration within the drilling fluid after the drilling fluid has been cycled through the wellbore. Ion concentration may be determined through chromatography, for example. The present disclosure describes some embodiments of mud gas analyzers capable of determining free and dissolved CO₂ quantities, as well as other gasses, within a drilling fluid.

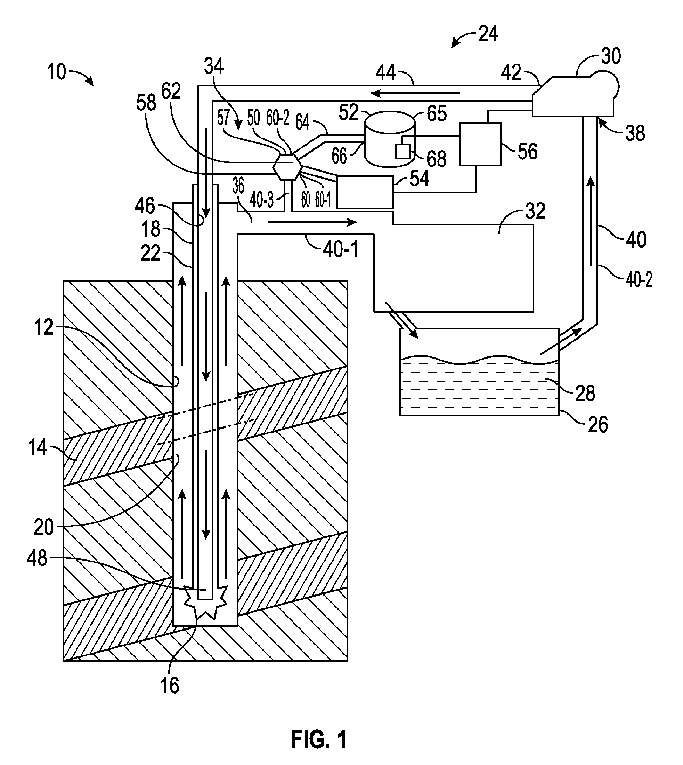

Referring now to the drawings, shown in Figure 1 is a well site 10 in accordance with the present disclosure. The well site 10 is provided with a wellbore 12 extending through a geological formation 14. The wellbore 12 is formed by advancing a drilling tool 16, connected to a drill string 18, through the geological formation 14. An annulus 20 is formed between an exterior surface 22 of the drill string 18 and the geological formation 14, thereby defining the wellbore 12.

In some embodiments, the drilling tool 16 may be conveyed on the drill string 18 through the geological formation 14 alone or among one or more measurement-while-drilling (MWD) drilling tools, logging-while-drilling (LWD) drilling tools, or other drilling tools that are known in the art. The drilling tool 16 may be driven by a rig (not shown) as it is advanced through the geological formation 14 forming the wellbore 12.

The well site 10 also includes a drilling mud system 24 in fluid communication with the drill string 18, drilling tool 16, and the annulus 20. The drilling mud system 24 may include one or more mud pit 26 containing a drilling mud 28; a pump 30 in fluid communication with the mud pit 26 and the wellbore 12; one or more shale shaker 32 in fluid communication with the wellbore 12 and the mud pit 26 and positioned proximate to the mud pit 26; and a mud gas analyzer 34 in fluid communication with the wellbore 12 and the one or more mud pit 26 and positioned in a fluid flow path 36 of the drilling mud 28 between the wellbore 12 and the one or more shale shaker 32.

The pump 28 has an inlet 38 receiving drilling mud 28 from the mud pit 26 via one of a plurality of flow lines 40, and an outlet 42 injecting the drilling mud 28 into the drill string 18 through a mud injection line 44. In some embodiments, the mud injection line 44 may be connected to the drill string 18 via a swivel (not shown) to permit the drill string 18 to be rotated via a kelly (not shown) relative to the mud injection line 44, thereby aiding the drilling tool 16 in forming the wellbore 12. The pump 30 circulates the drilling mud 28 through the fluid flow path 36 (shown by way of arrows in Figure 1) formed sequentially by the mud injection line 44, an inner bore (not shown) of the swivel, an inner bore 46 of the drill string 18, an inner bore 48 of the drilling tool 16, the annulus 20, a first flow line 40-1 of the plurality of flow lines 40, and a second flow line 40-2 of the plurality of flow lines 40.

The one or more shale shaker 32 is known in the art and can be implemented in a variety of manners. In general, the shale shaker 32 serves to remove cuttings from the drilling mud 28. In some embodiments, the shale shaker 32 may include a vibrating screen with openings through which the drilling mud 28, but not the cuttings, may pass.

As referenced above and as will be explained in more detail below, the drilling mud 28, received from the annulus 20, contains a mixture of gasses received from the geological formation 14, and residual gasses (also known as "recycled gas") that were entrained in the drilling mud 28 prior to injection into the drill string 18 via the pump 30. In some embodiments, the drilling mud 28 may undergo a process known in the art as "mud degassing" while the drilling mud 28 is passing through the shale shaker 32 and the mud pit 26 in which gasses entrained in the drilling mud 28 are transferred into the surrounding atmosphere. For example, free gasses, those not dissolved within the drilling mud 28, may be released through agitation of the drilling mud 28 within the shale shaker 32. In some embodiments, portions of the drilling mud 28 may be degassed by the mud gas analyzer 34, as will be explained in more detail below. In general, the drilling mud 28 that is injected into the drill string 18 after mud degassing contains some dissolved gasses and may retain some free gas not released during the mud degassing. Gasses from the geological formation 14 may be different than the recycled gas entrained in the drilling mud 28 as new portions of the geological formation 14 are being exposed to the drilling mud 28 during the drilling process. The gasses from the geological formation 14 and the recycled gas within the drilling mud 28 may be measured and quantified by the mud gas analyzer 34.

As shown in Figure 1, in some embodiments, the mud gas analyzer 34 is provided with a volumetric sampler 50, a free gas detection system 52 in fluid communication with the volumetric sampler 50, and a dissolved gas detection system 54 in fluid communication with the volumetric sampler 50. In some embodiments, the mud gas analyzer 34 may be provided with a computer system 56 electrically coupled to at least the free gas detection system 52 and the dissolved gas detection system 54 and optionally the volumetric sampler 50. As will be explained in more detail below, the volumetric sampler 50 may be configured to collect a known volume of a sample of drilling fluid, in this case the drilling mud 28. The sample of drilling fluid may be a known volume of the drilling mud 28 which has been transferred through the annulus 20 and at least a portion of the geological formation 14. The free gas detection system 52 may receive free gas, removed from the sample, from the volumetric sampler 50 and may determine a first quantity of the free gas. The dissolved gas detection system 54 may receive at least a portion of the known volume of the sample, after the free gas has been transferred to the free gas detection system 52. The dissolved gas detection system 54 may then detect a second quantity of a dissolved gas entrained in and subsequently released from the drilling mud 28. The computer system 56 may receive electrical signals from the free gas detection system 52 and the dissolved gas detection system 54 representative of the first and second quantities, respectively, and perform further processes on the electrical signals and/or present the electrical signals in a user perceivable format such as a graphical display on a screen of the first and second quantities.

As shown in Figure 1, the volumetric sampler 50 may be provided with a sampler housing 57, an inlet 58 defined by the sampler housing 57 and connected to the fluid flow path 36 to receive the drilling mud 28, one or more outlets 60 defined by the sampler housing 57, and one or more filtering apparatus 62 positioned proximate to the sampler housing 57 in a fluid flow path between the inlet 58 and the one or more outlets 60. In some embodiments, the inlet 58 of the volumetric sampler 50 may be in fluid communication with the fluid flow path 36, for example via a connection with the first flow line 40-1 such as a third flow line 40-3.

The volumetric sampler 50 may receive a known volume of a sample, in this case a known volume of the drilling mud 28, through the inlet 58, via the third flow line 40-3, and filter the known volume of the sample through the one or more filtering apparatus 62 to remove solids such as solid pollutants, rocks, mud additives, cuttings, and other solid pollutants carried by the drilling mud 28 from the wellbore 12 and the geological formation 14. The one or more filtering apparatus 62 may be selected from the group comprising a screen, a sieve, a cross flow filtration system, or other filtering apparatus or device suitable to prevent solid pollutants, of a predetermined size or larger, from passing through the one or more outlet 60 along with the known volume of the sample. In some embodiments, the volumetric sampler 50 may be included in a gas system (not shown) configured to degas the sample. The gas system (not shown) may be provided with the volumetric sampler 50 and a mechanical agitator, such as a shale shaker, or any other mechanism capable of releasing the free gas from the sample. After degassing the sample, the sample passes through a first outlet 60-1 of the one or more outlet 60 to the dissolved gas detection system 54. A free gas, an entrained gas released by degassing the sample, is transferred to the free gas detection system 52 through a second outlet 60-2 of the one or more outlet 60. The free gas may be representative of one or more predetermined gas to be measured, and may be a single gas, such as gaseous CO₂, or a combination of gasses. In some embodiments, the free gas may pass through a gas line 64 extending between the second outlet 60-2 of the volumetric sampler 50 and the free gas detection system 52.

The free gas detection system 52 may be provided with a detection chamber 65, an inlet 66 defined by the detection chamber 65 and in fluid communication with the volumetric sampler 50, and at least one sensor 68 connected to the detection chamber 65 and configured to determine a first quantity of the free gas, as will be explained in more detail below. The at least one sensor 68 may generate a first signal indicative of the first quantity of the free gas. In some embodiments, the at least one sensor 68 may be electrically coupled to the computer system 56 such that the first signal generated by the at least one sensor 68 may be transmitted to the computer system 56 for further processing and/or display in a user perceivable format. In some embodiments, the at least one sensor 68 may be implemented as an infrared gas sensor, a non-dispersive infrared absorption sensor, a pressure sensor, or other sensors capable of detecting the first quantity of the free gas.

In some embodiments, where the at least one sensor 68 is implemented as a non-dispersive infrared absorption sensor, the at least one sensor 68 may identify and quantify an amount of the free gas, for example CO₂, released from the drilling mud 28. The quantification of the amount of free gas released from the drilling mud may be represented by the first quantity. In these embodiments, the at least one sensor 68 may measure the free gas based on infrared absorption phenomena by the gas stream emerging from the gas line 64. In other embodiments, the at least one sensor 68 may also determine the first quantity by changes in pressure, temperature, or other metrics capable of indicating the first quantity of the free gas.

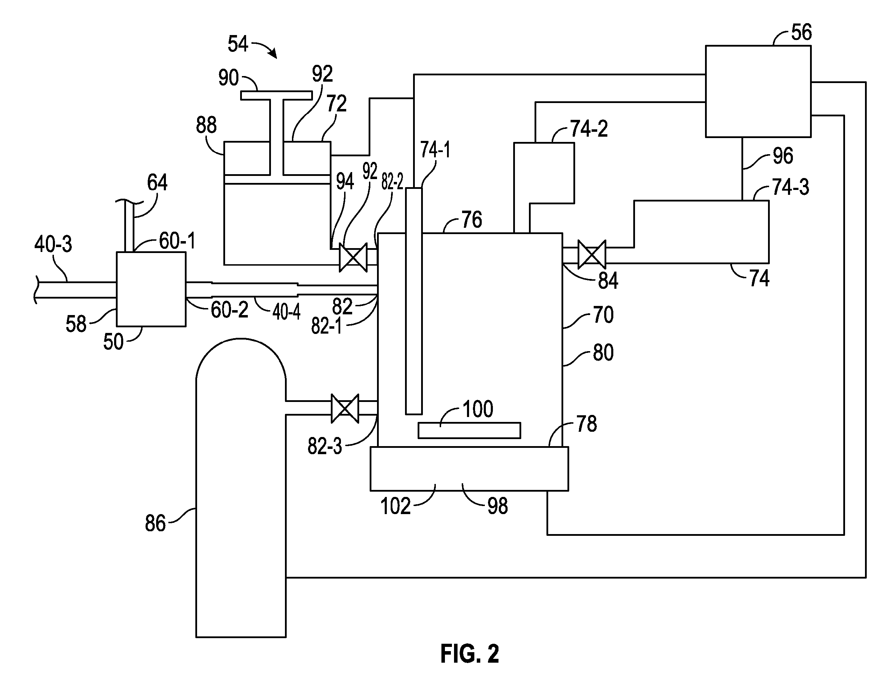

Referring now to Figures 1 and 2, the dissolved gas detection system 54 may include a reaction chamber 70 in fluid communication with the volumetric sampler 50, an acidification system 72 in fluid communication with the reaction chamber 70, and a sensor 74 connected to the reaction chamber 70. In some embodiments, the reaction chamber 70 is provided with a first end 76, a second end 78, one or more sidewalls 80 extending between the first end 76 and the second end 78, one or more inlet 82 defined by the one or more sidewalls 80, and one or more outlet 84 defined by the one or more sidewalls 80. In some embodiments, as shown in Figure 2, the one or more inlet 82 may be a plurality of inlets 82 with a first inlet 82-1, a second inlet 82-2, and a third inlet 82-3.

The reaction chamber 70 may be formed from steel, stainless steel, a polymer, or any other material suitable for use with substances common to a well servicing operation. The reaction chamber 70 may receive the known volume of the sample with the dissolved gas entrained therein via the first inlet 82-1, after the sample has been degassed in the volumetric sampler 50. As shown, the first inlet 82-1 may be in fluid communication with the second outlet 60-2 of the volumetric sampler 50 via a fourth flow line 40-4. The reaction chamber 70 may be in fluid communication with the acidification system 72 via the second inlet 82-2 to receive a known volume of an acid from the acidification system 72. In some embodiments, the reaction chamber 70 may be in fluid communication with an external gas supply 86 via the third inlet 82-3 to receive a portion of a gas from the external gas supply 86, as will be explained in more detail below.

The acidification system 72 may be provided with an acid tank 88 and an injection system 90 in fluid communication with the acid tank 88. The acid tank 88 may define an inlet 92 to receive volumes of an acid and an outlet 94 working in cooperation with the injection system 90 to discharge a known volume of acid into the reaction chamber 70. The injection system 90 may be connected to the acid tank 88 and be configured to inject the known volume of the acid, through the outlet 94, into the reaction chamber 70 through the second inlet 82-2 of the reaction chamber 70. In some embodiments, the injection system 90 may be provided as a mechanical piston, a pneumatic device, a pressurization system, a valve, or any other suitable mechanism. The injection system 90 may be configured to inject a predetermined amount of acid into the reaction chamber 70 based upon one or more signals received from the computer system 56. In some embodiments, the injection system 90 may include controls to adjust the predetermined amount of acid to ensure proper acidification of the sample within the reaction chamber 70 by matching the known volume of acid and the volume of the sample based a desired reaction stoichiometry.

Where the mud gas analyzer 34 is detecting the presence of CO₂, carbonate species present in the sample affect results of the acidification reaction. For example, CO₃²⁻ may be present as a reaction of CO₂ (aq) and the drilling mud 28. Ca(HCO₃)₂ may be present as a reaction of CO₂ (aq) with calcium carbonate, an additive used in some embodiments of the drilling mud 28. CaCO₃ may be present in solid form in the drilling mud 28, coming from rock surrounding the geological formation 14. (MgCa)(CO₃)₂ may be present in solid form in the drilling mud 28, originating in rock surrounding the geological formation 14 or as an additive in the drilling mud 28. In some embodiments, the acid may be hydrochloric acid, and may produce gaseous CO₂ from the above-referenced carbonates via the following reactions: CO₃²⁻ + 2HCl → CO_{2(g)} + H₂O + 2Cl⁻, Ca(HCO₃)₂ + 2HCl → 2CO_{2(g)} + 2H₂O + CaCl₂, CaCO₃ + 2HCl → CO_{2(g)} + CaCl₂ + H₂O, and (Mg.Ca)(CO₃)₂ + 4HCl → 2CO_{2(g)} + CaCl₂ + MgCl₂ + 2H₂O, respectively. The amounts of hydrochloric acid, or other acids, used may depend on the volume of the sample retrieved by the volumetric sampler 50 as well as the concentrations of the carbonates present within the sample. As such, in some embodiments, during the acidification process, acid volume may be calculated for a worst possible case, assuming 100% pure solid carbonates, to be sure that the acid volume is in excess of a minimum quantity to provide for an acidification reaction to occur.

The sensor 74 may be connected to the reaction chamber 70 and may detect the second quantity of the dissolved gas released from the known volume of the sample within the reaction chamber 70 during the acidification process. The sensor 74 may be selected from a group comprising a temperature sensor, a pressure sensor, a thermo conductive sensor, an infrared detection system, a combination thereof, or any other sensor capable of measuring the second quantity of the dissolved gas. The sensor 74 may be connected to the reaction chamber 70, housed within the reaction chamber 70, coupled to the outlet 84 of the reaction chamber, or otherwise positioned to measure the second quantity of the dissolved gas. In some embodiments, the sensor 74 may be electrically coupled to the computer system 56 and generate one or more second signal, such as an electrical signal, indicative of the second quantity of the dissolved gas. The sensor 74 may then transmit the one or more second signal to the computer system 56 via one or more connection 96 between the sensor 74 and the computer system 56.

The one or more connection 96 may be a wired or wireless connection capable of enabling transmission of the one or more electrical signal between the sensor 74 and the computer system 56.

In some embodiments, the sensor 74 may be implemented as one or more sensor 74. For example, in some embodiments similar to that shown in Figure 2, the sensor 74 is implemented as a first sensor 74-1, a second sensor 74-2, and a third sensor 74-3. The first sensor 74-1 may be a temperature sensor used for temperature compensation calculation. The second sensor 74-2 may be a pressure sensor capable of determining a pressure within the reaction chamber 70. In this embodiment, the first sensor 74-1 and the second sensor 74-2 may be used in cooperation, such that the first sensor 74-1 assists in temperature compensation calculation, where the second sensor 74-2 has been calibrated at a first temperature different than a second temperature at which the measurement of the dissolved gas takes place within the reaction chamber 70. In some embodiments, the second sensor 74-2, implemented as a pressure sensor, may measure the second quantity of the dissolved gas by sensing a pressure change within the reaction chamber 70.

The third sensor 74-3, in some embodiments, may be an infrared detection system connected to the outlet 84 of the reaction chamber 70. In some embodiments, the dissolved gas, released by the acidification process, may be transferred to the third sensor 74-3 by flashing the reaction chamber 70 with the gas from the external gas supply 86. For example, an inert gas stored within the external gas supply 86 may be injected into the reaction chamber 70 in sufficient volume to displace the dissolved gas released through the acidification process, forcing the dissolved gas to enter the infrared detection system of the third sensor 74-3. In some embodiments, the dissolved gas may be transferred to the third sensor 74-3 via suction, creating a lower pressure at the outlet 84 than is present in the reaction chamber 70. In some of these embodiments, the third sensor 74-3 may be implemented in a pump to create the lower pressure at the outlet 84.

As noted above, the first sensor 74-1, the second sensor 74-2, and the third sensor 74-3 may be electrically coupled to the computer system 56 and may generate one or more second signals indicative of the second quantity of the dissolved gas. In some embodiments, the first, second, and third sensors 74-1, 74-2, and 74-3 may generate one or more signals which are indicative of measurements and/or values other than the second quantity of the dissolved gas, such as temperature, pressure, fluid levels, etc. The first and second quantities may be used by the computer system 56, as will be described in more detail below, to estimate a total amount of one or more predetermined gas in the geological formation 14.

In some embodiments, the dissolved gas detection system 54 may be provided with an agitation system 98 connected to the reaction chamber 70. In some embodiments, the agitation system 98 may comprise a magnetic bar 100 positioned within the reaction chamber 70 and a magnetic stirrer 102 positioned proximate to the reaction chamber 70. As shown, the magnetic stirrer is positioned proximate to the second end 78 of the reaction chamber 70 such that the magnetic stirrer 102 may be operated to cause the magnetic bar 100 to agitate at least a portion of a contents of the reaction chamber 70, such as the acid and the sample. In some embodiments the agitation system 98 may be implemented as a mechanical agitation system, a sonic agitation system, combinations thereof, or other agitation system capable of agitating at least a portion of the contents of the reaction chamber 70.

Although not shown, the mud gas analyzer 34 may be provided with a second volumetric sampler, a second free gas detection system, and a second dissolved gas detection system in fluid communication with the mud pit 26, which may operate similarly to the volumetric sampler 50, the free gas detection system 52, and the dissolved gas detection system 54, respectively, described above. The second volumetric sampler may operate to collect, filter, and degas a known volume of the drilling mud 28 to serve as a reference sample. The second free gas detection system may operate to measure a third quantity of free gas entrained in the reference sample. The second free gas detection system may then generate a third signal indicative of the third quantity and transmit the third signal to the computer system 56. The second dissolved gas detection system may operate to measure a fourth quantity of the dissolved gas in the reference sample. The second dissolved gas detection system may then generate a fourth signal indicative of the fourth quantity and transmit the fourth signal to the computer system 56. The third and fourth quantities may be used, by the computer system 56, to estimate and/or adjust the estimated total amount of the one or more predetermined gas in the geological formation 14.

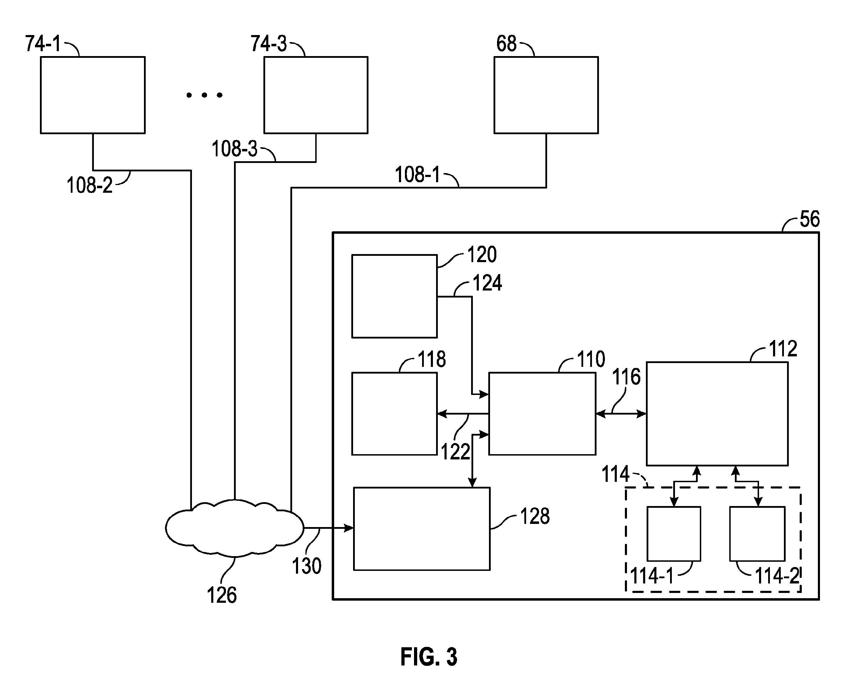

Referring now to Figures 2 and 3, in some embodiments, the computer system 56 is connected to the at least one sensor 68 of the free gas detection system 52 and the sensor 74 of the dissolved gas detection system 54 to analyze the free and dissolved gasses of the known volume of the sample, such as the drilling mud 28. For example, the computer system 56 may be connected to the at least one sensor 68 and the sensors 74-1 and 74-3 via communication channels 108-1, 108-2, and 108-3, which may be wired or wireless communication channels. In some embodiments, the computer system 56 may be connected to the volumetric sampler 50 and the acidification system 72 to control the intake of the known volume of the sample and the acidification of the sample after the sample has been degassed. In some embodiments using the external gas supply 86, the external gas supply 86 may be connected to the computer system 56 to control flashing of the reaction chamber 70. The computer system 56, in some embodiments, may also be connected to the agitation system 98 to control agitation of at least a portion of the sample within the reaction chamber 70. In some embodiments, the computer system 56 may also be connected to the pump 30 to control transfer of the drilling mud 28 from the mud pit 26 to the wellbore 12. The computer system 56 may comprise a processor 110, a non-transitory processor readable medium 112, and processor executable instructions 114 stored on the non-transitory processor readable medium 112. In some embodiments, the computer system 56 may receive flow/depth information from an operator, a flow/depth providing service, or a sensor.

The processor 110 may be implemented as a single processor or multiple processors working together or independently to execute the processor executable instructions 114 described herein. Embodiments of the processor 110 may include a digital signal processor (DSP), a central processing unit (CPU), a microprocessor, a multi-core processor, an application specific integrated circuit, and combinations thereof. The processor 110 is coupled to the non-transitory processor readable medium 112. The non-transitory processor readable medium 112 can be implemented as RAM, ROM, flash memory or the like, and may take the form of a magnetic device, optical device or the like. The non-transitory processor readable medium 112 can be a single non-transitory processor readable medium, or multiple non-transitory processor readable medium functioning logically together or independently.

The processor 110 is coupled to and configured to communicate with the non-transitory processor readable medium 112 via a path 116 which can be implemented as a data bus, for example. The processor 110 may be capable of communicating with an input device 118 and an output device 120 via paths 122 and 124, respectively. Paths 122 and 124 may be implemented similarly to, or differently from path 116. For example, paths 122 and 124 may have a same or different number of wires and may or may not include a multidrop topology, a daisy chain topology, or one or more switched hubs. The paths 116, 122 and 124 can be a serial topology, a parallel topology, a proprietary topology, or combination thereof. The processor 110 may be further capable of interfacing and/or communicating with one or more network 126, via a communications device 128 and a communications link 130 such as by exchanging electronic, digital and/or optical signals via the communications device 128 using a network protocol such as TCP/IP. The communications device 128 may be a wireless modem, digital subscriber line modem, cable modem, network bridge, Ethernet switch, direct wired connection or any other suitable communications device capable of communicating between the processor 110 and the network 126.

It is to be understood that in certain embodiments using more than one processor 110, the processors 110 may be located remotely from one another, located in the same location, or comprising a unitary multicore processor (not shown). The processor 110 is capable of reading and/or executing the processor executable instructions 114 and/or creating, manipulating, altering, and storing computer data structures into the non-transitory processor readable medium 112.

The non-transitory processor readable medium 112 stores processor executable instructions 114 and may be implemented as random access memory (RAM), a hard drive, a hard drive array, a solid state drive, a flash drive, a memory card, a CD-ROM, a DVD-ROM, a BLU-RAY, a floppy disk, an optical drive, and combinations thereof. When more than one non-transitory processor readable medium 112 is used, one of the non-transitory processor readable mediums 112 may be located in the same physical location as the processor 110, and another one of the non-transitory processor readable mediums 112 may be located in a location remote from the processor 110. The physical location of the non-transitory processor readable mediums 112 may be varied and the non-transitory processor readable medium 112 may be implemented as a "cloud memory," i.e. non-transitory processor readable medium 112 which is partially or completely based on or accessed using the network 126. In some embodiments, the non-transitory processor readable medium 112 stores a database accessible by the computer system 56.

In some embodiments, the input device 118 transmits data to the processor 110, and can be implemented as a keyboard, a mouse, a touch-screen, a camera, a cellular phone, a tablet, a smart phone, a PDA, a microphone, a network adapter, a camera, a scanner, the one or more sensor 68, the sensor 74, and combinations thereof. The input device 118 may be located in the same location as the processor 110, or may be remotely located and/or partially or completely network-based. The input device 118 communicates with the processor 110 via path 122.

In some embodiments, the output device 120 transmits information from the processor 110 to a user, such that the information can be perceived by the user. For example, the output device 120 may be implemented as a server, a computer monitor, a cell phone, a tablet, a speaker, a website, a PDA, a fax, a printer, a projector, a laptop monitor, and combinations thereof. The output device 120 communicates with the processor 110 via the path 124.

The network 126 may permit bi-directional communication of information, data, and/or electrical signals between the processor 110 and the mud gas analyzer 34. The network 126 may interface with the processor 110 in a variety of ways, such as by optical and/or electronic interfaces, and may use a plurality of network topographies and protocols, such as Ethernet, TCP/IP, circuit switched paths, file transfer protocol, packet switched wide area networks, and combinations thereof. For example, the one or more network 126 may be implemented as the Internet, a LAN, a wide area network (WAN), a metropolitan network, a wireless network, a cellular network, a GSM-network, a CDMA network, a 3G network, a 4G network, a satellite network, a radio network, an optical network, a cable network, a public switched telephone network, an Ethernet network, and combinations thereof. The network 126 may use a variety of network protocols to permit bi-directional interface and communication of data, information, and/or electrical signals between the processor 110 and the network 126.

In one embodiment, the processor 110, the non-transitory processor readable medium 112, the input device 118, the output device 120, and the communications device 128 may be implemented together as a smartphone, a PDA, a tablet device, such as an iPad, a netbook, a laptop computer, a desktop computer, or any other computing device.

The non-transitory processor readable medium 112 may store the processor executable instructions 114, which may comprise a mud gas analysis program 114-1. The non-transitory processor readable medium 112 may also store other processor executable instructions 114-2 such as an operating system and application programs such as a word processor or spreadsheet program, for example. The processor executable instructions for the mud gas analysis program 114-1 and the other processor executable instructions 114-2 may be written in any suitable programming language, such as C++, C#, or Java, for example.

The mud gas analysis program 114-1 may have processor executable instructions which enable control of the mud gas analyzer 34, the mud pump 30, other equipment at the well site 10, and combinations thereof. The mud gas analysis program 114-1 may have processor executable instructions for receiving the sequences of first and second signals from the mud gas analyzer 34. In some embodiments, the mud gas analysis program 114-1 may have processor executable instructions for receiving the sequences of third and fourth signals from the mud gas analyzer 34. To control the mud gas analyzer 34, the mud gas analysis program 114-1, may allow for manual control for reading the first, second, third, and fourth, signals of the mud gas analyzer 34. In some embodiments, the mud gas analysis program 114-1 may also allow for setting the flow rate of the drilling mud 28, as measured by a flow meter. The mud gas analysis program 114-1 may have processor executable instructions, for interpreting and/or outputting information received from the one or more sensor 68 and the sensor 74 of the mud gas analyzer 34 to create user perceivable outputs, in the form of reports, waveforms, or display screens for example to provide a user with the information indicative of the free and dissolved gasses of the drilling mud 28 transported through the wellbore 12 past, and in some cases through, the geological formation 14, as well as the free and dissolved gasses of the drilling mud 28 prior to being transported through the wellbore 12.

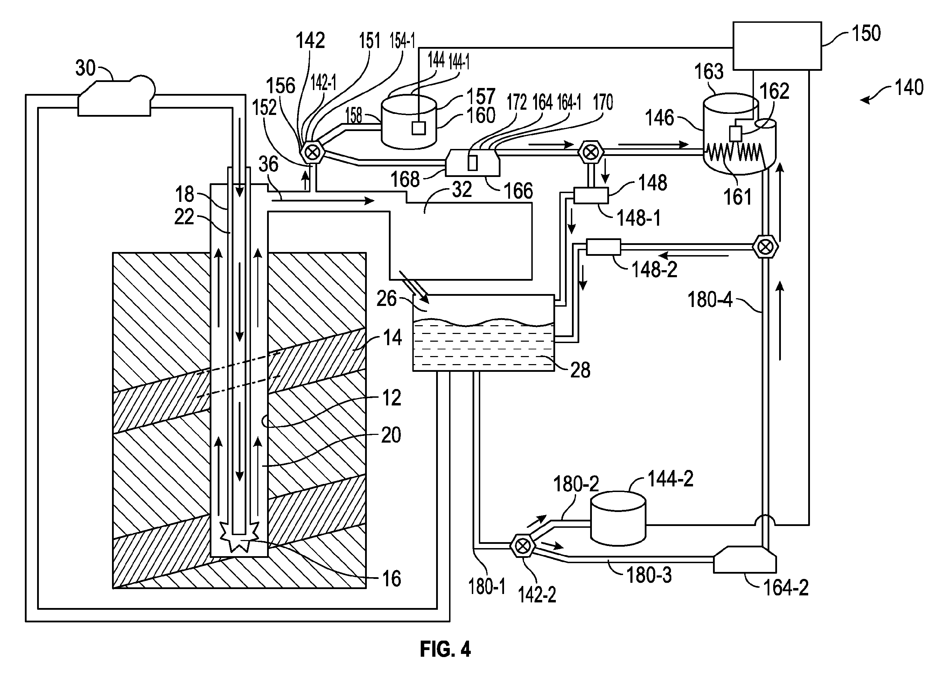

Referring now to Figure 4, therein shown is a mud gas analyzer 140, according to some embodiments of the present disclosure. The mud gas analyzer 140 may be positioned at or near the wellbore 12 and connected to the drill string 18 via the drilling mud system 24. The mud gas analyzer 140 comprises a volumetric sampler 142 configured to collect a known volume of a sample of drilling fluid having a free gas and a dissolved gas, a free gas detection system 144 in fluid communication with the volumetric sampler 142, an ionic chromatography system 146 in fluid communication with the volumetric sampler 142, and one or more sensor 148 in fluid communication with the ionic chromatography system 146. In some embodiments, the mud gas analyzer 140 may include a computer system 150 connected to the free gas detection system 144, the ionic chromatography system 146, and the one or more sensor 148.

The volumetric sampler 142 may be implemented similarly or the same as the volumetric sampler 50, and may be provided with sampler housing 151, an inlet 152 defined by the sampler housing 151 and connected to the fluid flow path 36 of the drilling mud 28, one or more outlets 154 defined by the sampler housing 151, and one or more filtering apparatus 156 positioned proximate to the sampler housing 151 in a fluid flow path between the inlet 152 and the one or more outlets 154. In some embodiments, the inlet 152 of the volumetric sampler 142 may be in fluid communication with the fluid flow path 36, for example by a connection with the first flow line 40-1.

The volumetric sampler 142 may receive a known volume of a sample of the drilling mud 28 through the inlet 152, filter the sample, and release the free gas entrained within the known volume of the sample to the free gas detection system 144. The one or more filtering apparatus 156 may remove solid pollutants such as rocks, mud additives, cuttings and other solid pollutants carried by the sample which are below a predetermined size. The one or more filtering apparatus 156 may be a screen, a sieve, a cross flow filtration system, or other filtering device. The volumetric sampler 142 may be included in a gas system or may be provided with an agitator capable of releasing the free gas from the sample, in a process called degassing. After the sample is degassed, the free gas may pass through a first outlet 154-1 of the one or more outlet 154 to the free gas detection system 144. The degassed sample may pass through a second outlet 154-2 toward the ionic chromatography system 146.

The free gas detection system 144 receives the free gas from the volumetric sampler and determines a quantity of the free gas. The free gas detection system 144 may be implemented similarly or the same as the free gas detection system 52 and may include a housing 157, an inlet 158 defined by the housing 157, and one or more sensor 160. The inlet 158 may receive the free gas from the volumetric sampler 142. The one or more sensor 160 may be capable of determining a first quantity of the free gas. The one or more sensor 160 may be implemented similarly to the at least one sensor 68 and may be electrically coupled to the computer system 150 such that a first signal, generated by the one or more sensor 160 upon detection of the first quantity of the free gas, may be transmitted to the computer system 150 for further processing. The one or more sensor 160 may be an infrared gas sensor, a non-dispersive infrared absorption sensor, a pressure sensor, a combination thereof, or any other sensor capable of detecting the first quantity of the free gas. In some embodiments, the free gas is CO₂ and the first quantity is a quantity of CO₂ released from the sample.

The ionic chromatography system 146 may be capable of determining a concentration of predetermined ions within the known volume of the sample. In some embodiments, the ionic chromatography system 146 is provided with one or more separation column 161, one or more sensors 162 in fluid communication with the one or more separation column 161, and a housing 163 storing the one or more separation column 161 and the one or more sensors 162. The one or more sensors 162 may be capable of identifying the predetermined ions and the respective concentrations of the predetermined ions. The ionic chromatography system 146 may be implemented as a high performance liquid chromatography system such as a Dionex® UltiMate® 300 LC system (Dionex®, Sunnyvale, CA., USA.) using an ICE-AS6 column (Dionex®, Sunnyvale, CA., USA.), or any suitable liquid or ionic chromatography system capable of determining a presence and concentration of predetermined ions within the known volume of the sample. After being introduced to the ionic chromatography system 146, the sample may pass into the one or more separation column 161. The one or more separation column 161 separates the sample and may pass the sample to the one or more sensors 162 in fluid communication with the one or more separation column 161. The one or more sensors 162 may then detect the presence of the predetermined ions within the sample and the concentrations of the predetermined ions. In some embodiments, the one or more sensor 162 may generate a second signal indicative of the concentration of the predetermined ions and transmit the second signal to the computer system 150. In these embodiments, the second signal may be analyzed by the computer system 150 to determine the quantity of dissolved gas entrained within the sample.

The one or more sensors 162 may be implemented as thermal conductivity detectors, flame ionization detectors, electrochemical sensors, or any other suitable sensors capable of determining presence and concentration of the predetermined ions, such as carbonate ions, for example. In some embodiments, the predetermined ions may be carbonate ions such as bicarbonate (HCO₃⁻) and carbonate (CO₃²⁻).

In some embodiments, the ionic chromatography system 146 may also include a filtration system 164. The filtration system 164 may be integrated into the ionic chromatography system 146 and positioned within the housing 163. In these embodiments, the filtration system 164 may be operated automatically to filter the sample as the sample is received into the ionic chromatography system 146. In some embodiments, the filtration system 164 may be implemented separately from the ionic chromatography system 146. After degassing, the sample is transferred to the ionic chromatography system 146, but the sample may be filtered prior to being processed through the ionic chromatography system 146. The sample may be filtered by the filtration system 164 until the sample reaches a predefined clarity of filtrate for the sample, prior to being introduced into the ionic chromatography system 146.

In some embodiments, the filtration system 164 may be dialysis filtration. In some embodiments, the filtration system 164 may be implemented as a cross flow filtration system. In these embodiments, the filtration system 164 may be held at positive pressure, relative to a filtrate side. The sample may be caused to flow tangentially across the surface of the filter, rather than into the filter. A portion of the sample which is smaller than a pore size of the filter may then pass as a filtrate. The filtration system 164 may include a filtration chamber 166, an inlet 168 defined by the filtration chamber 166, an outlet 170 defined by the filtration chamber 166, and a filter 172 positioned within the filtration chamber 166. The filtration system 164, in some embodiments, may also include a delivery system (not shown) such as a pump. The delivery system may be provided outside of the filtration chamber 166 and transfer the sample from the outlet 170 of the filtration system 164 to the ionic chromatography system 146. In some embodiments, the filtration system 164 may be provided with a second pump inverting pressure on the filter 172. The second pump may enable cleaning of the filter 172 by reversing pressure on the sample or other fluid passing through the filter 172.

The one or more sensor 148 may be implemented as one or more of a pH sensor, a conductivity sensor, and a redox electrode. In some embodiments, the one or more sensor 148 may analyze the second quantity of the dissolved gas within the sample. In some embodiments, the one or more sensor 148 may analyze one or more qualities or quantities of the sample, aside from the first quantity of the free gas and the second quantity of the dissolved gas. For example, the one or more sensor 148 may measure the pH level of the sample, after the sample has been filtered by the filtration system 164. In some embodiments, the pH level of the sample may be used to establish solubility levels for CO₂ and carbonate species in drilling fluids. Additionally the pH level may be used for optimization of final results within the computer system 150.

The computer system 150 may be implemented similarly to or the same as the computer system 56. The computer system 150 may have a processor, a non-transitory processor readable medium electrically connected to the processor, and processor executable instructions stored on the non-transitory processor readable medium. The computer system 150 may be electrically coupled to at least one of the one or more sensor 160 of the free gas detection system 144, the one or more sensors 162 of the ionic chromatography system 146, and the one or more sensor 148 via a plurality of communication links. The communication links may be wireless or wired communication links capable of transmitting electrical signals, information, and/or data to and from the computer system 150. The processor executable instructions stored on the non-transitory processor readable medium of the computer system 150 may cause the processor to execute logic to cause the processor to receive the first signal, indicative of the first quantity of the free gas, from the free gas detection system 144; receive the second signal, from the ionic chromatography system 146 and the one or more sensor; and estimate a total amount of one or more certain type of gas in a formation based on the first quantity and the second quantity.

As show in Figure 4, in some embodiments, the volumetric sampler 142 is a first volumetric sampler 142-1, the free gas detection system 144 is a first free gas detection system 144-1, the sensor 148 is a first sensor 148-1, and the filtration system 164 is a first filtration system 164-1. In these embodiments, the mud gas analyzer 140 may further be provided with a second volumetric sampler 142-2, a second free gas detection system 144-2, a second sensor 148-2, and a second filtration system 164-2. The second volumetric sampler 142-2 may be implemented similarly to the first volumetric sampler 142-1. The second free gas detection system 144-2 may be implemented similarly to the first free gas detection system 144-1. The second sensor 148-2 may be implemented similarly to the first sensor 148-1. The second filtration system 164-2 may be implemented similarly to the first filtration system 164-1.

As shown, the second volumetric sampler 142-2 may be in fluid communication with the mud pit 26 containing the drilling mud 28 via a first flow line 180-1 connecting an outlet of the mud pit 26 and an inlet of the second volumetric sampler 142-2. In some embodiments, the volumetric sampler 142-2 may sample and receive a known volume of the drilling mud 28 from the mud pit 26, prior to the drilling mud 28 being cycled through the wellbore 12. The known volume of the drilling mud 28 may serve as a reference sample, used in some embodiments to determine free and dissolved gasses present in the drilling mud 28. The free and dissolved gasses in the reference sample may be used to adjust the estimation of the mud gas analyzer 140 for the one or more predetermined gasses present in the geological formation 14. The second volumetric sampler 142-2 may collect the known volume of the reference sample, filter the known volume of the reference sample, and release the free gas entrained within the known volume of the reference sample, similar to the process discussed for the volumetric samplers 50 and 142. The second volumetric sampler 142-2 may then release the free gas to the second free gas detection system 144-2 via a second flow line 180-2 connecting an outlet of the second volumetric sampler 142-2 and an inlet of the second free gas detection system 144-2.

The second free gas detection system 144-2 may receive the free gas from the second volumetric sampler 142-2 and determine a third quantity of the free gas released from the reference sample. The second free gas detection system 144-2 may then generate a third signal, indicative of the third quantity of the free gas released from the reference sample, and transmit the third signal to the computer system 150. The computer system 150 may analyze the third signal to determine the quantity of free gas released from the reference sample. In some embodiments, the third quantity of the free gas of the reference sample may be compared with the first quantity of the free gas of the sample as part of the process of estimating the total amount of the one or more predetermined gas within the geological formation 14.

The second filtration system 164-2 may receive the reference sample from the second volumetric sampler 142-2, after the reference sample has been degassed to release the free gas from the reference sample. The second filtration system 164-2 may filter the reference sample until the reference sample reaches a predefined clarity of filtrate to use as a reference filtrate for the sample prior to being introduced into the ionic chromatography system 146.

The ionic chromatography system 146 may receive the known volume of reference filtrate, the filtered reference sample, via a fourth flow line 180-4 extending between an outlet of the second filtration system 164-2 and an inlet of the ionic chromatography system 146. The ionic chromatography system 146 may pass the reference sample into the one or more separation column 161 to separate the reference sample and transfer the separated reference sample to the one or more sensors 162 in fluid communication with the one or more separation column 161. The one or more sensors 162 may detect the presence of the predetermined ions within the reference sample and the concentrations of the predetermined ions. In some embodiments, the one or more sensor 162 may generate a fourth signal indicative of the concentration of the predetermined ions and transmit the fourth signal to the computer system 150. In these embodiments, the fourth signal may be analyzed by the computer system 150 to determine a fourth quantity of the dissolved gas entrained within the reference sample.

In some embodiments, the second sensor 148-2 may analyze a fourth quantity of the dissolved gas within the sample. In some embodiments, the second sensor 148-2 may analyze one or more qualities or quantities of the reference sample, aside from the third quantity of the free gas and the fourth quantity of the dissolved gas of the reference sample. For example, the second sensor 148-2 may measure the pH level, conductivity, oxidation, or other qualities of the reference sample after the reference sample has been filtered by the filtration system.

In some embodiments, the mud gas analyzer 34 or the mud gas analyzer 140 may be used to measure and sum quantities of free gas and dissolved gas within a sample of the drilling mud 28. In some embodiments, a method for measuring free and dissolved gas within a sample of the drilling mud 28 may be performed by collecting, by the volumetric sampler 50 or 142, a known volume of a sample from a drilling fluid exiting a wellbore penetrating a formation. The sample has a free gas and a dissolved gas. The free gas detection system 52 or 144 may measure a first quantity of the free gas entrained in the sample. The free gas may be measured after being released by the volumetric sampler 50 or 142 or the free gas detection system 52 or 144. The method may further be performed by transferring the sample to the dissolved gas detection system 54 or the ionic chromatography system 146. In some embodiments, transportation of the sample may include filtering the sample through the filtering apparatus 62 or 156 or the filtering system 164.

Where transferred to the dissolved gas detection system 54, the dissolved gas detection system 54 may measure a second quantity of the dissolved gas in the sample. After being transferred to the dissolved gas detection system 54, in some embodiments, the sample is acidified to release the dissolved gas entrained within the sample, after degassing the sample to release the free gas. Acidifying the sample may be performed by adding a known volume of acid to the sample. The volume of the acid and the type of acid may be predetermined based on reaction stoichiometry specific to the dissolved gas to be released from the sample. A concentration of the dissolved gas, released from the acidification of the sample, may then be determined using the dissolved gas detection system 54, for example by the use of the sensor 74 or a combination of the sensors 74-1, 74-2, and 74-3.

Where transferred to the ionic chromatography system 146, the sample may be filtered, by the filtration system 164, to create a known volume of a filtrate. The known volume of the filtrate may be introduced to the ionic chromatography system 146 and a concentration of predetermined ions within the known volume of the filtrate may be measured. The method may further be performed by determining the second quantity of the dissolved gas based on the concentration of the predetermined ions.

Whether transferred to the dissolved gas detection system 54 or the ionic chromatography system 146, the computer system 56 or 150 may be used to estimate a total amount of one or more predetermined gas in a formation based on the first and second quantities of the free gas and the dissolved gas, respectively.

In some embodiments, the method may further be performed by collecting, by the volumetric sampler 50 or 142, a known volume of a reference sample of drilling fluid prior to injecting the drilling fluid into the wellbore. The known volume of the reference sample has a free gas and a dissolved gas. The reference sample may include a known volume of the drilling mud 28 sampled prior to injection into the wellbore. The free gas detection system 52 or 144 may measure a third quantity of the free gas entrained in the reference sample. The reference sample may be transferred to the dissolved gas detection system 54 or the ionic chromatography system 146 after the free gas entrained within the sample is released. Transportation of the reference sample may include filtering the sample through the filtering apparatus 62 or 156 or the filtering system 164. Where transferred to the dissolved gas detection system 54, a fourth quantity of the dissolved gas may be measured in the reference sample by the dissolved gas detection system 54. A computer system 56 or 150 may be used to adjust the estimated total amount of the one or more predetermined gas in the formation based on the third and fourth quantities.

Although a few embodiments of the present disclosure have been described in detail above, those of ordinary skill in the art will readily appreciate that many modifications are possible without materially departing from the teachings of the present disclosure. Accordingly, such modifications are intended to be included within the scope of the present disclosure as defined in the claims.

## Claims

1. A method, comprising:
collecting, by a volumetric sampler, a known volume of a sample from drilling fluid exiting a wellbore penetrating a formation, the sample having a free gas and a dissolved gas;
measuring, by a free gas detection system, a first quantity of the free gas entrained in the sample;
transferring the sample to a dissolved gas detection system after the free gas entrained in the sample is released;
measuring a second quantity of the dissolved gas in the sample by the dissolved gas detection system; and
estimating a total amount of one or more predetermined gas in a formation based on the first and second quantities.

2. The method of claim 1, wherein the volumetric sampler is included in a gas system configured to degas the sample and determine a quantity of the free gas released from the sample.

3. The method of claim 1 or 2, wherein transferring the sample further comprises filtering the sample through a filtering apparatus to remove solids from the sample prior to measuring the second quantity of the dissolved gas.

4. The method of claim 3, wherein the filtering apparatus is selected from a group comprising a screen, a sieve, and a cross flow filtration system.

5. The method according to anyone of claims 1 to 4, wherein the first quantity is indicative of substantially all of the free gas entrained in the sample.

6. The method according to anyone of claims 1 to 5, wherein the second quantity is indicative of substantially all dissolved of the dissolved gas in the sample.

7. The method according to anyone of claims 1 to 6, further comprising:
collecting, by a volumetric sampler, a known volume of a reference sample of drilling fluid prior to injecting the drilling fluid into the well bore, the known volume of the reference sample having a free gas and a dissolved gas;
measuring, by a free gas detection system, a third quantity of the free gas entrained in the reference sample;
transferring the reference sample to a dissolved gas detection system after the substantially all of free gas entrained in the sample is released;
measuring a fourth quantity of the dissolved gas in the reference sample by the dissolved gas detection system; and
wherein estimating the total amount of the one or more predetermined gas includes adjusting the estimated total amount of the one or more predetermined gas in the formation based on the third and fourth quantities.

8. The method according to anyone of claims 1 to 7, wherein measuring the second quantity in the dissolved gas detection system further comprises:
acidifying the sample to release the dissolved gas entrained within the sample, after degassing the sample to release the free gas; and
determining a concentration of the dissolved gas released from the acidification of the sample.

9. The method according to anyone of claims 1 to 8, wherein acidifying the sample is performed by adding a known volume of acid to the sample, and wherein the volume of acid is predetermined based on reaction stoichiometry.

10. The method according to anyone of claims 1 to 9, wherein the dissolved gas detection system includes a filtration system and an ionic chromatography system, and wherein measuring the second quantity of the dissolved gas further comprises:
filtering, through the filtration system, the sample to create a known volume of a filtrate;
introducing the known volume of the filtrate to the ionic chromatography system;
measuring, by the ionic chromatography system, a concentration of predetermined ions within the known volume of the filtrate; and
determining the second quantity of the dissolved gas based on the concentration of the predetermined ions.

11. The method of claim 10, wherein the filtration system is a cross flow filtration system.

12. A mud gas analyzer, comprising:
a volumetric sampler having a sampler housing, an inlet defined by the sampler housing, one or more outlet defined by the sampler housing, and one or more filtering apparatus positioned proximate to the sampler housing in a fluid flow path between the inlet and the one or more outlets;
a free gas detection system having a detection chamber, an inlet defined by the detection chamber and in fluid communication with certain of the one or more outlets of the volumetric sampler, and at least one sensor connected to the detection chamber;
an ionic chromatography system having a housing, one or more separation column positioned within the housing and in fluid communication with the volumetric sampler, and one or more sensor positioned within the housing and in fluid communication with the one or more separation column; and
one or more sensor, in fluid communication with the ionic chromatography system.

13. The mud gas analyzer of claim 12, wherein the volumetric sampler includes filtering apparatus which is selected from a group comprising a screen, a sieve, and a cross flow filtration system.

14. The mud gas analyzer of claim 12 or 13, wherein the ionic chromatography system further includes a filtration system.

15. The mud gas analyzer of claim 12, 13 or 14, wherein the one or more sensor is selected from a group comprising a pH sensor, a conductivity sensor, and a redox electrode.

16. The mud gas analyzer according to anyone of claims 12 to 15, further comprising a computer system, electrically coupled to the free gas detection system and at least one of the ionic chromatography system and the one or more sensor, the computer system including a processor adapted to execute logic to cause the processor to receive a first signal, indicative of a first quantity, from the free gas detection system, receive a second signal, indicative of a second quantity, from at least one of the ionic chromatography system and the one or more sensor, and estimate a total amount of one or more certain type of gas in a formation based on the first quantity and the second quantity.
